# EUROPEAN PATENT SPECIFICATION

(11) **EP 2 841 126 B1**
(45) Date of publication and mention of the grant of the patent: **15.06.2016**
(21) Application number: 13725789.5
(22) Date of filing: 15.04.2013
(51) Int. Cl.: A61M 1/16, B01D 63/02, A61M 1/36

(54) **PROCESS AND APPARATUS FOR REMOVING CONDENSATION FROM A DEVICE FOR THE EXTRACORPOREAL HEMATIC TREATMENT FOR THE REMOVAL OF CARBON DIOXIDE**
VERFAHREN UND VORRICHTUNG ZUM ENTFERNEN VON KONDENSWASSER AUS EINER VORRICHTUNG ZUR EXTRAKORPORALEN BLUTBEHANDLUNG ZUR ENTFERNUNG VON KOHLENDIOXID
PROCÉDÉ ET APPAREIL D'ÉLIMINATION DE LA CONDENSATION À PARTIR D'UN DISPOSITIF POUR LE TRAITEMENT HÉMATIQUE EXTRACORPOREL POUR L'ÉLIMINATION DE DIOXYDE DE CARBONE

(30) Priority: 27.04.2012 IT BS20120074
(43) Date of publication of application: 04.03.2015
(73) Proprietor: Estor SpA, 20016 Pero (MI) (IT)
(72) Inventor: CARONNA, Marco, 20016 Pero (MI) (IT); GUADAGNI, Gualtiero, 20016 Pero (MI) (IT); FIORE, Gianfranco, 20016 Pero (MI) (IT)
(74) Representative: Sangiacomo, Ines
(86) International application number: PCT/IB2013/052978
(87) International publication number: WO 2013/160799

(56) References cited:
- EP-A1- 1 941 919
- EP-A2- 0 183 250
- EP-A2- 1 043 036
- WO-A1-2011/053414
- US-A- 4 196 075

## Description

### Field of the Invention

The present invention relates to a process for the removal of condensation from a device, such as an oxygenator of the type used for the extracorporeal treatment of blood, particularly for the removal of carbon dioxide from and/ or the oxygenation of the blood, and an apparatus for carrying out the afore said treatment.

### State of the Art

The extracorporeal treatment of blood for the removal of carbon dioxide from and/ or the oxygenation of the blood involves substantially taking blood from a patient, making it to flow externally of a bundle of internally hollow capillaries which are passed through by a working gas (sweep gas), usually medical-grade air, and then re-infusing it into the patient via a central venous catheter. This treatment makes use of a device such as an oxygenator, also referred to as a CO₂ removal cartridge, which is provided with an inlet and an outlet for a flow of venous blood from and to the patient, an inlet for receiving a flow of working gas from a special dispensing system which is usually available in the health-care facility, and an outlet for the working gas. In the oxygenator, the working gas exchanges with the blood, through a separation member formed as a microporous membrane comprising hollow capillaries through which the working gas flows and intended to be passed through by gases such as carbon dioxide, oxygen and water vapor as a result of the concentration gradient existing between the blood and the working gas.

The water vapor passing through the microporous membrane causes the formation of condensation droplets which tend to clog the lumina of the hollow capillaries constituting the membrane itself, thereby reducing the flow of gas passing through them. Therefore, the membrane should be periodically cleaned to remove the condensation.

Document WO 2011/053414 describes an apparatus for removing condensation from an oxygenator, comprising a suction pump located downstream of the oxygenator in order to make the working gas to flow through the oxygenator itself, a condensation collecting unit in fluidic communication with the oxygenator and the suction pump, and a mechanism for controlling the flow of working gas, said mechanism having an opened position which allows the gas to pass from the oxygenator to the collecting unit, and a closed position in which the flow of gas from the oxygenator to the collecting unit is prevented. When the control mechanism is in the opened position, the suction pump draws the working gas from the oxygenator and the collecting unit, and when the control mechanism is in the closed position, the suction pump generates a negative pressure in the collecting unit. The sudden flow rate increase of the working gas passing through the oxygenator causes the detachment of the condensation droplets from the lumina of the hollow capillaries of the microporous membrane. This cleaning process is often referred to with the phrase *causing the oxygenator to cough.*

This approach is complex as it requires that the oxygenator gas compartment continuously works under vacuum, thereby requiring the use of a suction pump which is relatively large in size and therefore expensive and cumbersome. Moreover, the flow of working gas is interrupted for a certain period of time in order to generate the vacuum required to cause the oxygenator to cough. Indeed, there is a need to minimize this period of time in order to simplify the removal of condensation from the hollow capillaries as much as possible. Moreover, the flow of working gas is only possible when the suction pump is operating, the flow of working gas when this pump is shut off would be zero.

### Summary of the Invention

Accordingly, the main aim of the present invention is to provide a process and apparatus which can overcome the drawbacks of traditional approaches by quickly providing an effective patency of the lumina of the hollow capillaries of the microporous membrane while the oxygenator is operating and without the aid of a suction pump to maintain a constant vacuum in the capillaries of the oxygenator membrane.

Therefore, in a first aspect, the present invention relates to a process for removing condensation from the lumina of the capillaries of a microporous membrane provided in an oxygenator passed through by a flow of venous blood according to claim 1.

In particular, the process comprises the steps of:
a) feeding a flow of working gas, at a first pressure value, into the capillaries of the oxygenator membrane for the removal of carbon dioxide from and/ or the oxygenation thereof;
b) assigning a constant value to or detecting the value of the pressure of the flow of venous blood entering or leaving or existing within the oxygenator;

Advantageously the method comprises the further step of:
c) producing at least one pressure peak in the flow of working gas fed to the oxygenator, with the provision that the maximum value of the pressure peak caused in the flow of working gas is lower than the assigned or detected pressure value of the flow of venous blood.

For example, the working gas is a medical-grade air of the type usually available in health-care facilities.

Compared to known solutions, the method according to the present invention can provide an effective purging of hollow capillaries of the microporous membrane while the oxygenator is operating or rather the blood of the patient is being treated.

Document WO 2011/053414 describes a technical prejudice. On page 3, lines 20-23, it is stated that, if the pressure of the working gas entering the oxygenator is increased to cause it to cough, it is not possible to prevent gas pressure from exceeding the pressure of the venous blood existing within the oxygenator itself, with the risk of formation of emboli in the blood.

Instead, the Applicant found that this drawback can be circumvented by producing rapid, controlled pressure increases in the flow of working gas fed to the oxygenator, i.e. positive peaks whose duration is less than 3 seconds, preferably less than 0.5 seconds, while actively maintaining the maximum pressure of the peak at a value lower than the pressure of the venous blood, which is in turn either detected by sensors or assumed to be constant and equal to a preset threshold value.

As will be clarified in the description below, the phrase *actively maintaining* the pressure peak of the working gas below a threshold means using suitable tools to intercept the flow of working gas and prevent its absolute pressure from exceeding the detected or assigned threshold value of the flow of venous blood.

Preferably, the pressure value of the flow of venous blood is obtained by detecting the corresponding instantaneous value of the venous blood in the oxygenator or coming out therefrom, otherwise such a threshold value is assigned to be constant over time. Practically, there are provided two operating modes. In a first mode, the pressure of the flow of venous blood is not known a priori, and its detection is performed, for example, by a sensor that is in the apparatus and intended to measure pressures in the extracorporeal circuit.

In a second mode, the pressure of the flow of venous blood within the oxygenator is not known, and the pressure value of the working gas is maintained below the minimum threshold value usually employed with extracorporeal blood circuits, for example below a value equal to 30 mmHg for a veno-venous circuit.

Preferably, the pressure peak produced in the flow of working gas fed to the oxygenator is a pressure increase to at least twice said first pressure value and then a pressure decrease to the first pressure value. The pressure increase and decrease occur within a time lapse of less than 3 seconds, preferably of less than 0.5 seconds.

Advantageously, the pressure peak in the flow of working gas is achieved by increasing and subsequently decreasing the instantaneous flow rate of the working gas to the capillaries. The increase and decrease of the instantaneous flow rate occur within a time lapse of less than 3 seconds, preferably of less than 0.5 seconds.

Preferably step c) is feedback carried out based on the detected pressure value of the flow of venous blood. In other words, when the pressure value of the flow of venous blood is known, the pressure peak in the flow of working gas is adjusted correspondingly so as to not exceed that value; in this case, the pressure value of the flow of venous blood is detected at least once, but preferably continuously during the blood treatment. On the contrary, in the second operating mode, the threshold pressure is assigned a constant value over time and the pressure peak in the flow of working gas is adjusted correspondingly so as to not exceed that value. In the preferred embodiment, step c) is performed at regular time intervals during the extracorporeal blood treatment of step a). For example, pressure peaks are produced in the flow of working gas every 2 minutes or every 6 minutes or every 10 minutes, etc. The duration of the time intervals can be varied even during the treatment; for example, the first peak is cased after 5 minutes, the second peak after 10 minutes, etc.

If necessary, prior to step a), there is provided a further step of heating the working gas to a temperature close or equal to the temperature of the flow of venous blood, for example 37°C.

Preferably, the process according to the present invention comprises the additional steps of:
d) detecting the concentration of carbon dioxide in the flow of working gas coming out from the oxygenator, and
e) calculating the amount of carbon dioxide extracted from the blood flow crossing the oxygenator based on the concentration value detected in the step d) multiplied by the corresponding value of the flow rate of the working gas.

Preferably, step e) is repeated over time to monitor the amount of carbon dioxide extracted from the flow of blood throughout the blood treatment.

If the efficiency is unsatisfactory, it is possible either to produce a pressure peak in the flow of working gas or to increase the peak frequency so as to purge the lumina of the hollow capillaries of the microporous membrane.

Preferably, the working gas passing through the capillaries of the membrane flows in counter-current with respect to the flow of venous blood passing through the oxygenator.

In a second aspect, the present invention relates to an apparatus for the extracorporeal treatment of blood according to claim 9.

Particularly, the apparatus comprises an oxygenator provided with a microporous membrane comprising bundles of hollow capillaries, means for feeding a flow of venous blood at a first pressure value through the membrane externally of the hollow capillaries, and means for feeding, preferably in counter-current, a flow of working gas at a first pressure value into said hollow capillaries.

Advantageously, the apparatus comprises a control unit for controlling the feeding means of the working gas programmed to produce at least one pressure peak in the flow of working gas fed to the oxygenator with the provision that the maximum value of the pressure peak caused in the flow of working gas is lower than the value of a threshold pressure which is either equal to the pressure of the flow of venous blood in the oxygenator or coming out therefrom, and/or equal to an assigned constant value.

Preferably, the means for feeding the flow of venous blood through the microporous membrane comprise, in turn, means for detecting the pressure value of the flow of venous blood. For example, such means may comprise pressure sensors inserted either immediately downstream of the oxygenator or within it. Alternatively or in addition, the control unit comprises means for inputting the constant value assigned to the threshold pressure. For example, for a veno-venous extracorporeal circuit, the constant value is equal to 30 mmHg. Preferably, the means for feeding the flow of working gas comprise an inflow line of the gas from an outer source, for example available in a health-care facility, to the oxygenator. The control unit comprises a proportional electrovalve for the interception of the inflow line. The proportional valve can be operated to produce a rapid peak of flow rate, and consequently a rapid peak of pressure in the flow of working gas, while preventing the maximum pressure of the peak from exceeding the threshold value, e.g. a value determined by feedback based on the pressure value detected from or assigned to the flow of venous blood within the oxygenator.

Preferably, the control unit is programmed to control the opening and closing of the proportional electrovalve so as to cause each peak of flow rate, and consequently each peak of pressure in the flow of working gas, within a time lapse of less than 3 seconds, preferably of less than 0.5 seconds.

Preferably, the control unit is programmed to control the opening and closing of the proportional electrovalve so as to cause several peaks of pressure in the flow of working gas at prearranged and/ or programmable time intervals, as described above with respect to the method according the present invention.

The detection means comprise at least one sensor configured to detect the instantaneous pressure value of the flow of venous blood within the oxygenator or leaving/entering it. The detection means allow the process of the invention to be carried out in the first operating mode.

In one embodiment, the apparatus further comprises at least one heater of the working gas arranged to bring the temperature of the working gas to a value close or equal to the temperature value of the flow of venous blood, for example 37°C.

Preferably, the apparatus comprises means for taking, as needed, at least one flow rate of working gas coming out from the oxygenator, i.e. a flow rate of gas which has interacted with the flow of blood, thus having a concentration of CO₂ which is higher than that in the working gas, and at least one sensor for detecting the concentration of carbon dioxide in the working gas coming out from the oxygenator. The apparatus further comprises a computing unit programmed to calculate the amount of carbon dioxide extracted from the blood flow passing through the oxygenator based on the carbon dioxide concentration value detected by the sensor. For example, the sensor for detecting the concentration of carbon dioxide is an infrared sensor.

Preferably, the condensation formed in the oxygenator and removed from the lumina of the hollow capillaries of the microporous membrane as a result of positive pressure peaks produced in the flow of working gas, is preferably collected by gravity at the bottom of the oxygenator and removed therefrom, again by gravity, possibly using a bag connected to the bottom of the oxygenator.

Unlike the solution described in WO 2011/053414, the apparatus according to the present invention is free of a suction pump serving for maintaining the oxygenator under a vacuum. Indeed, such a pump is not required, resulting in the obvious advantages that the apparatus is simplified and its overall dimensions and costs are reduced.

### Detailed Description of the Invention

Further characteristics and advantages of the present invention will be more evident from a review of the following specification of a preferred, but not exclusive, embodiment, shown for illustration purposes only and without limitation, with the aid of the attached drawings, in which:
- Figure 1 is a schematic view of the apparatus according to the present invention;

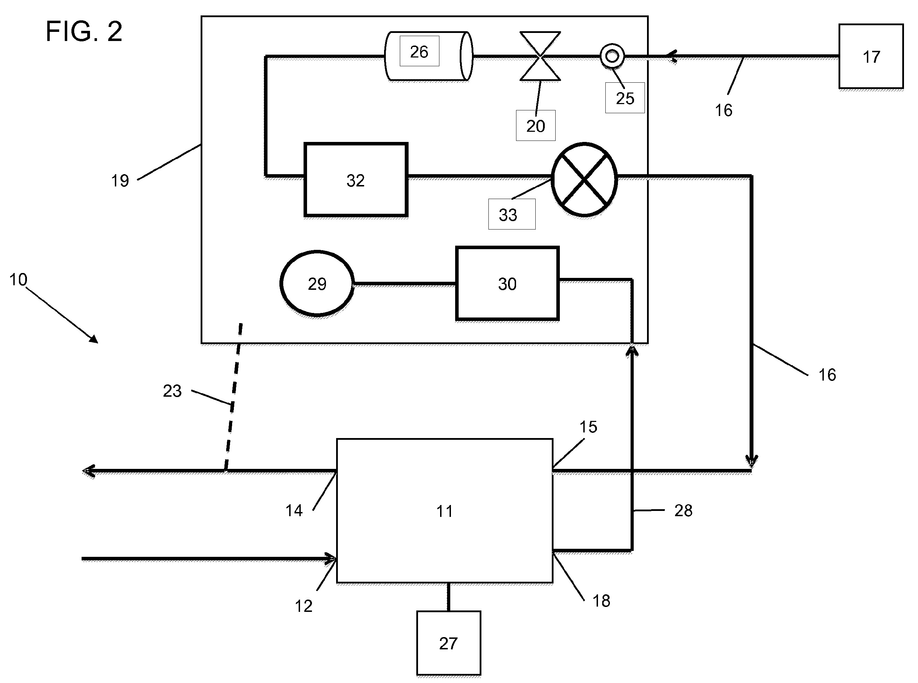

- Figure 2 is a block diagram of the apparatus of Figure 1;

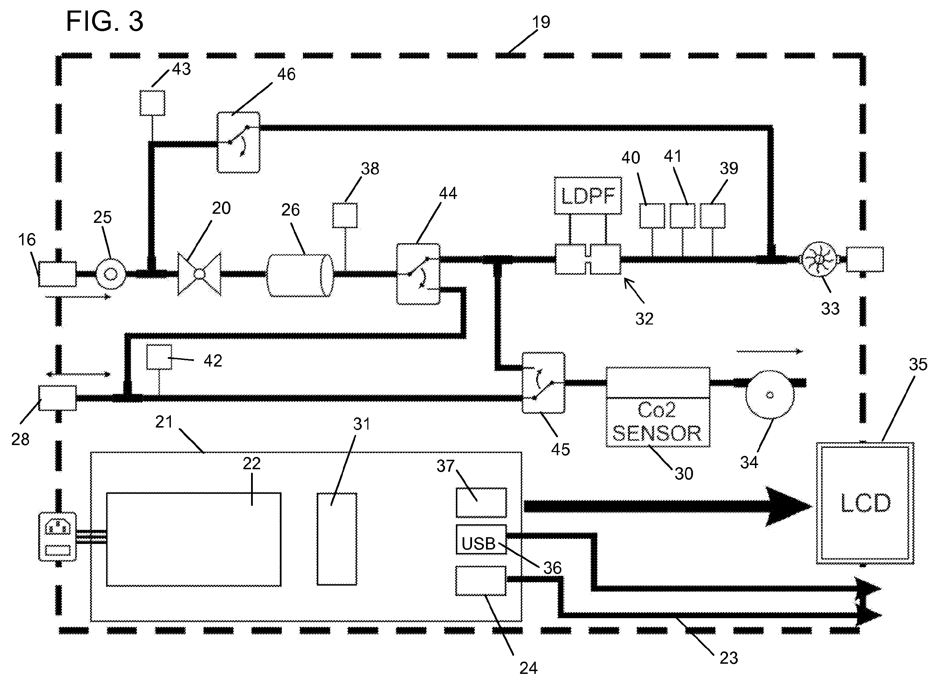

- Figure 3 is a block diagram of the control unit alone;
- Figure 4 shows an example of the time course of the venous pressure and working gas pressure values.

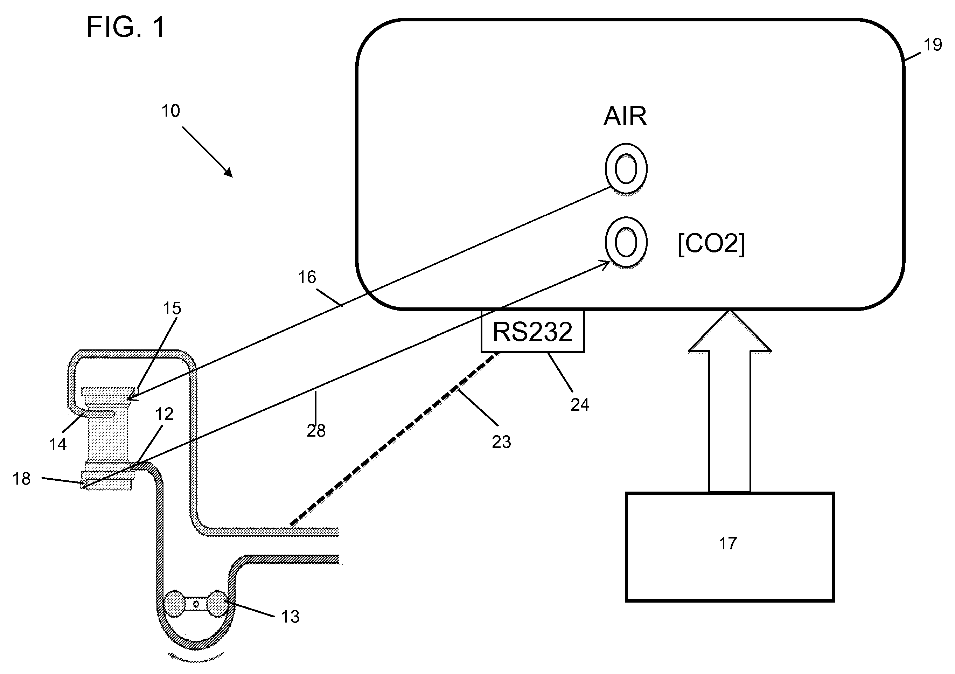

In Figure 1 an example of an apparatus 10 for the extracorporeal treatment of blood is illustrated, comprising an oxygenator **11** having a membrane - not shown - therein of the type having blood-impermeable and gas-permeable hollow capillaries. The oxygenator has a first opening **12** for a flow intake of venous blood from a patient, for example via a first central venous catheter provided with a peristaltic pump **13,** a second opening **14** for the coming out of the blood flow to the patient, for example via a second central venous catheter, an inlet port **15** for receiving a flow of working gas which is fed through a inflow line **16** from a dispensing system **17,** and an outlet port **18** for expelling the exhaust working gas which has interacted with the blood within the oxygenator. The working gas, which can be oxygen or medical-grade air, is fed into the hollow capillaries of the membrane whereas the blood is caused to flow externally of them. In this way, the oxygen content of the working gas diffuses through the capillaries to enrich the venous blood. On the contrary, part of the carbon dioxide present at high concentrations in the incoming blood diffuses in the working gas which is then expelled out from the oxygenator through the outlet port **18**. The flow of venous blood comes out from the oxygenator at a first pressure value **Pᵥ,** while the flow of working gas is fed to the oxygenator at a first pressure value **P1_{g}**.

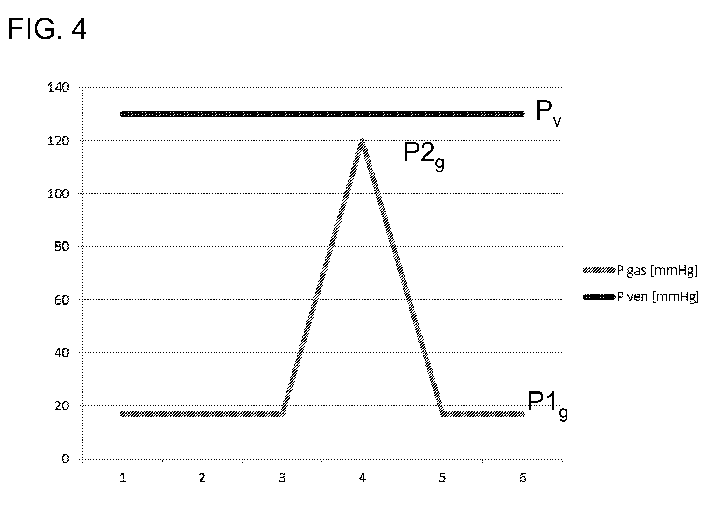

The apparatus **10** further includes a control unit **19** located on the working gas inflow line **16** and interposed between the dispensing system **17** and the oxygenator **11**. The main function of the control unit is to produce at least one pressure peak in the flow of working gas fed to the oxygenator, with the provision that the maximum value **P2_{g}** of the pressure peak produced in the working gas is lower than a threshold pressure value which can be either equal to the pressure value **Pᵥ** of the venous blood existing within the oxygenator or coming out therefrom, as shown in Figure 4, or equal to a constant value which is assigned before the treatment. This serves to prevent air bubbles from entering the flow of blood returning to the patient, which may cause embolisms.

In order to produce the above said pressure peak, the control unit is provided with a proportional intercepting electrovalve **20** operatively located on the inflow line. The proportional electrovalve can be appropriately driven to increase and subsequently decrease its opening and opening speed so as to produce a rapid increase of the instantaneous flow rate of the gas directed towards the capillaries, followed by a rapid decrease thereof, thereby producing a corresponding pressure peak in the flow of working gas.

The proportional electrovalve **20** is driven by a controlling electronics **21** provided on the control unit. It comprises a microcontroller **22** which operates to read the threshold pressure value and increase and then decrease the opening of the proportional electrovalve in order to produce multiple pressure peaks **P2_{g}** in the flow of working gas at prearranged and/or programmable time intervals while maintaining the value **P2_{g}** under the threshold pressure value. Each peak in the gas flow, and thus the corresponding increase and subsequent decrease in the electrovalve opening, has a duration of less than 3 seconds, preferably of less than 0.5 seconds. The pressure peaks in the flow of working gas can be caused at regular intervals, for example every 2 minutes or every 6 minutes or every 10 minutes, etc., or at varying intervals during the treatment.

The threshold pressure value can be assigned a constant value, for example a value equal to or lower than 30 mmHg for an extracorporeal veno-venous circuit, or it can be determined by feedback based on the pressure value **Pᵥ** detected in the flow of venous blood existing within the oxygenator or coming out therefrom. In this second case, the apparatus includes a feedback line **23** through which the control unit **19** receives, as an input via an interface port **24**, the instantaneous pressure value **Pᵥ** of the flow of venous blood as detected by appropriate sensors. The interface port can be of the RS232 type. The control unit may be provided with input means to allow a user to establish the timing of the pressure peaks and/ or to input the threshold value as necessary.

In order to further increase the apparatus safety, the apparatus can also be provided with means to always maintain the pressure of the blood flow within the oxygenator at a value equal to or greater than 30 mmHg, thereby satisfying the requirement that the pressure value of the blood flow is always greater than the maximum peak pressure value.

Furthermore, a flow-restricting orifice **25** is located upstream of the proportional electro-valve, preferably within the control unit, in order to provide a first restriction in the flow rate of the working gas from the dispensing system. Furthermore, a flow-restricting orifice **25** can be programmed not only to produce the pressure peaks, but also to adjust the flow rate of the treatment gas leaving the flow restrictor **25** in such a way as to bring it to a value consistent with the treatment itself, for example a value in the range from 0 to 30 liters per minute.

In order to decrease the formation of water vapor within the capillaries, the apparatus may further comprise at least one heater **26** configured in such a way as to bring the temperature of the working gas to a value close or equal to the temperature value of the flow of venous blood, for example in the range from 35°C to 37°C. The working gas thus heated also helps to remove the condensation which gradually builds up on the capillaries. Advantageously, such a heater is provided on the control unit.

The condensation removed from the lumina of the hollow capillaries of the microporous membrane as a result of the positive pressure peaks caused in the flow of working gas, is collected by gravity at the bottom of the oxygenator and removed - again by gravity - therefrom. Advantageously, a collection bag 27 can be arranged in fluidic connection with the bottom of the oxygenator itself where the condensation is collected.

The apparatus may further comprise a return line **28** and a suction pump **29** to take at least a flow rate of the working gas coming out from the oxygenator after its interaction with the blood flow, and to convey it into the control unit **19**. Additionally, the control unit is provided with at least one sensor **30** for detecting the concentration of carbon dioxide in the working gas entering and coming out from the oxygenator from the return line, and a computing unit **31** programmed to calculate the amount of carbon dioxide extracted from the blood flow passing through the oxygenator. The amount of extracted carbon dioxide is calculated by multiplying the value of the carbon dioxide concentration in the working gas coming out from the oxygenator by the flow rate value of the working gas; such a flow rate value is measured by means of a flow-meter **32** provided in the control unit on the inflow line **16** downstream of the proportional electrovalve **20**. The suction pump **29**, which may be a diaphragm pump, takes a minimum portion of the volume of working gas coming out from the oxygenator; the optimal flow rate is in the range of 100-500 ml/min. The sensor **30** for detecting the concentration of carbon dioxide is, for example, an infrared sensor.

Advantageously, the flow-meter **32** can be associated with a flow visual indicator **33** to check whether the unit is operating or not from the outside of the control unit. If the amount of carbon dioxide is found to be unsatisfactory, i.e. below a certain value, the controlling electronics can be programmed to automatically cause a peak in the flow of working gas or else to automatically increase the frequency of the peaks.

A gas-suction pump **34** can be provided downstream of the sensor **30** for detecting the concentration of carbon dioxide in the working gas.

The control unit may further comprise a display **35**, for example an LCD-type display, to visualize the measured parameters, i.e. the amount of carbon dioxide removed from the blood and the flow rate value of the working gas entering the oxygenator, by means of indicators or charts. Advantageously, the display is of a *touch-screen* type so that a user can input the threshold pressure value as necessary and establish the timing at which the pressure peaks occur sequentially. Alternatively or in addition, the control unit can be connected to a personal computer - not shown - for exporting the measured data and programming the control unit; a USB port **36** may also be provided for the connection with external devices.

Therefore, in summary, the controlling electronics **21** has the tasks of supervising all the components of the system, reading the threshold pressure value, driving the proportional electrovalve so as to cause the pressure peaks, establishing the timing of pressure peaks and measuring the concentration of carbon dioxide in the flow of leaving working gas, performing the calculations required to obtain the amount of carbon dioxide removed from the blood and driving the display, if any, or exporting the measured data. Furthermore, the controlling electronics **21** may be provided with an internal memory card **37** for the data storage.

Numerals **38** and **39** denote temperature sensors, numerals **40** to **43** denote pressure sensors, and numerals **44**, **45** and **46** denote three-way electrovalves, which components may be provided on the control unit for ensuring its proper operation and monitoring sensitive parameters.

The above described apparatus can be used to carry out the process for removing condensation from the capillaries of the microporous membrane of the oxygenator according to the invention.

Concretely, such a process comprises the steps of:
feeding a flow of working gas to the oxygenator, at a first pressure value **P1_{g},** for the removal of carbon dioxide from the blood and/ or the oxygenation thereof;
assigning a constant value to, or detecting, the pressure value **Pᵥ** of the flow of venous blood;
causing at least one pressure peak **P2_{g}** in the flow of working gas feeding the oxygenator, taking care of observing the condition **P1_{g}** < **P2_{g} < Pᵥ.**

The pressure peak **P2_{g}** is obtained by increasing and subsequently decreasing the instantaneous flow rate of the gas flow.

According to the process, the pressure **Pᵥ** of the flow of venous blood is obtained by detecting the instantaneous pressure value of the flow of venous blood within the oxygenator, otherwise it is a constant threshold value assigned downstream of the oxygenator; for example, for a veno-venous extracorporeal circuit, such a threshold value can be maintained preferably at a value lower than or equal to 30 mmHg.

In another process aspect, the pressure peak causing a pressure peak **P2_{g}** consists of a pressure increase of at least the double with respect to the first pressure value **P1_{g}** followed by a decrease of pressure, the latter returning back to the first pressure value **P1_{g}**; the pressure increase and decrease happen in a lapse of time shorter than 3 s, preferably shorter than 0.5 seconds.

Preferably, the step of causing at least one pressure peak **P2_{g}** in the flow of working gas is feedback carried out based on the detected pressure value of the flow of venous blood.

Advantageously, the step of producing at least one pressure peak **P2_{g}** in the flow of working gas is carried out at regular time intervals during the extracorporeal blood treatment.

Furthermore, if necessary, prior to the step of feeding a flow of working gas to the oxygenator at a first pressure value **P1_{g}**, there is provided a further step of increasing the temperature of the working gas to a value close or equal to the temperature value of the flow of venous blood; preferably, this value will be in the range from 35°C to 37°C.

Eventually, the process may additionally comprise the further steps of:
detecting the concentration of carbon dioxide in the flow of working gas coming out from, and possibly entering, the oxygenator, and calculating the amount of carbon dioxide extracted from the blood flow passing through the oxygenator by multiplying the concentration values detected at the previous step by the corresponding flow rate values of the working gas.

Preferably, the calculation of the amount of carbon dioxide extracted from the blood flow is repeated over time to monitor the amount of carbon dioxide extracted from the blood flow throughout the blood treatment.

Furthermore, if the above said amount is found to be unsatisfactory, it is possible either to produce a peak in the flow rate, and consequently a pressure peak in the flow of working gas, or to increase the peak frequency so as to purge the lumen of hollow capillaries of the microporous membrane.

## Claims

1. Process for removing condensation from an oxygenator crossed by a flow of venous blood and provided with a hollow - capillary membrane, the process comprising the step of:
a) feeding a flow of working gas to said hollow capillaries, at a first pressure value (P1_{g}), for the removal of carbon dioxide from the blood and/ or the oxygenation thereof;
b) assigning a constant value to, or detecting, the pressure value (Pᵥ) of the flow of venous blood;
c) causing at least one pressure peak (P2g) in the flow of working gas fed to the hollow capillaries, taking care of observing the condition P1_{g} < P2_{g} < Pᵥ.

2. Process according to claim 1, wherein said pressure value (Pᵥ) of the venous flow is obtained by detecting the instantaneous value of the flow pressure of venous blood in the oxygenator, or which is coming out therefrom, or else said pressure value (Pᵥ) of the venous flow is assigned to a constant value.

3. Process according to claim 2, wherein said pressure value (Pᵥ) of flow of venous blood is assigned to a constant value equal to 30 mmHg.

4. Process according to any one of the preceding claims, wherein said pressure peak (P2g) consists of a pressure increase of at least the double with respect to the first pressure value (P1_{g}) followed by a decrease of pressure, the latter returning back to the first pressure value (P1_{g}), and wherein the pressure increase and decrease happen in a lapse of time shorter than 3 s.

5. Process according to any one of the preceding claims, wherein the step c) is obtained increasing and subsequently decreasing the instantaneous flow rate of the working gas fed to the hollow capillaries.

6. Process according to claim 2, wherein the step c) is feedback carried out based on the detected pressure value (Pᵥ) of the flow of venous blood.

7. Process according to any one of the preceding claims, wherein the step c) is carried out at steady time intervals during the extracorporeal hematic treatment of the step a).

8. Process according to any one of the preceding claims, wherein, when needed, before the step a) the further step of increasing the temperature of the working gas up to a value next, or equal, to the temperature value of the flow of venous blood is provided.

9. Process according to any one of the preceding claims, comprising the further steps of:
d) detecting the concentration of carbon dioxide in the flow of working gas coming out from the oxygenator, and
e) monitoring the amount of carbon dioxide extracted from the blood flow crossing the oxygenator based on the concentration values detected in the step d), multiplied by the corresponding values of the flow rate of the working gas.

10. Apparatus (10) for an extracorporeal hematic treatment, comprising an oxygenator (11) provided with a hollow - capillary membrane, means for feeding a flow of venous blood through the oxygenator at a first pressure value (Pᵥ), and means for feeding a flow of working gas through said hollow capillaries at a first pressure value (P1_{g}), **characterized by** comprising a control unit (19) of the feeding means of the working gas programmed to cause at least one pressure peak (P2g) in the flow of working gas fed to the oxygenator, observing the condition P1_{g} < P2_{g} < Pᵥ.

11. Apparatus according to claim 10, wherein said feeding means of the flow of venous blood comprise in their turn means for detecting the first pressure value (Pᵥ) of the flow of venous blood or means for assigning a constant value to the pressure value (Pᵥ) of the flow of venous blood.

12. Apparatus according to claim 10 or 11, wherein said feeding means of the flow of working gas comprise an inflow line (16) of working gas from an outer source (17) towards said hollow capillaries and the control unit (19) comprises a proportional electrovalve (20) for the interception of said inflow line which can be operated to cause said at least one pressure peak (P2g) in the flow of working gas, increasing and decreasing the instantaneous flow rate of the working gas fed to the hollow capillaries.

13. Apparatus according to claim 12, wherein the control unit (19) is programmed to control the opening and the closing of said proportional electrovalve (20) to cause said at least one pressure peak (P2g) in the flow of working gas in a lapse of time shorter than 3 s.

14. Apparatus according to claim 12 or 13, wherein the control unit (19) is programmed to control the opening and the closing of said proportional electrovalve (20) to cause several pressure peaks (P2g) in the flow of working gas at prearranged and/ or programmable time intervals.

15. Apparatus according to any one of the claims 10 to 14, wherein the control unit (19) comprises means programmable for assigning a constant value to the pressure value (Pᵥ) of flow of venous blood, or else said detecting means comprise at least one sensor arranged to detect the instantaneous pressure value (Pᵥ) of the flow of venous blood inside the oxygenator or coming out therefrom.

16. Apparatus according to any one of the claims, further comprising at least one heater (26) of the working gas arranged to increase the temperature of working gas to a value next, or equal, to the temperature value of the flow of venous blood.

17. Apparatus according to any one of the claims 10 - 16, comprising means for taking at least one flow rate of working gas coming out from the oxygenator after the interaction thereof with the blood flow, and at least one sensor (30) for detecting the concentration of carbon dioxide in the working gas fed to the oxygenator and in the working gas coming out from the oxygenator, and a computing unit (31) programmed to calculate the amount of carbon dioxide taken from the blood flow crossing the oxygenator as a difference between the values of carbon dioxide concentration detected by said sensor.

## Patentansprüche

1. Verfahren zum Entfernen von Kondensat aus einem von einem venösen Blutstrom durchströmten und mit einer Hohl-Kapillarmembrane versehenen Oxygenator, wobei das Verfahren folgende Schritte umfasst:
a) Zuführen eines Arbeitsgasstromes mit einem ersten Druckwert (P1_{g}) zu den Hohlkapillaren, um Kohlendioxid aus dem Blut zu entfernen und/oder für seine Oxygenation;
b) Zuordnen oder Erfassen eines konstanten Wertes zum Druckwert (Pᵥ) des venösen Blutstromes;
c) Bewirken zumindest eines Druckspitzenwertes (P2_{g}) in dem den Hohlkapillaren zugeführten Arbeitsgasstrom, unter Einhaltung der Bedingung P1_{g} < P2_{g} < Pᵥ.

2. Verfahren nach Anspruch 1, wobei der Druckwert (Pᵥ) des venösen Blutstroms durch Ermitteln des augenblicklichen Stromwertes des im Oxygenator befindlichen oder aus ihm ausströmenden venösen Blutes erhalten wird oder der Druckwert (Pᵥ) des venösen Stromes einem konstanten Wert zugeordnet wird.

3. Verfahren nach Anspruch 2, wobei der Druckwert (Pᵥ) des venösen Blutstroms einem konstanten Wert von 30 mmHg zugeordnet wird.

4. Verfahren nach einem der vorangehenden Ansprüche, wobei der Druckspitzenwert (P2g) aus einem zumindest doppelten Druckanstieg gegenüber dem ersten Druckwert (P1_{g}) besteht, gefolgt von einem Druckabfall, der auf dem ersten Druckwert (P1_{g}) zurückkehrt, und wobei Druckanstieg bzw. Druckabfall in einer Zeitdauer, die kürzer als 3 Sekunden ist erfolgen.

5. Verfahren nach einem der vorangehenden Ansprüche, wobei der Schritt c) durch Erhöhung und anschließende Erniedrigung der momentanen Flussrate des in den Hohlkapillaren zugeführten Arbeitsgases erreicht wird.

6. Verfahren nach Anspruch 2, wobei der Schritt c) aufgrund des erfassten Druckwertes (Pᵥ) des venösen Blutstromes mit Rückkopplung durchgeführt wird.

7. Verfahren nach einem der vorangehenden Ansprüche, wobei der Schritt c) bei einem stationären Zeitintervall während der extrakorporalen Blutbehandlung des Schrittes a) durchgeführt wird.

8. Verfahren nach einem der vorangehenden Ansprüche, wobei nach Bedarf vor dem Schritt a) ein weiterer Schritt des Temperaturanstiegs des Arbeitsgases bis zu einem Wert, der nahe oder gleich dem Temperaturwert des venösen Blutstromes ist, durchgeführt wird.

9. Verfahren nach einem der vorangehenden Ansprüche, weiter umfassend die Schritte;
d) Ermitteln der Konzentration von Kohlendioxid in dem aus dem Oxygenator auskommenden Arbeitsgasfluss, und
e) Überwachen der Kohlendioxidmenge, die aus dem Blutfluss extrahiert wurde, welcher durch den Oxygenator durchströmt ist, aufgrund der im Schritt d) ermittelten Konzentrationswerte, multipliziert mit den entsprechenden Werten der Flussrate des Arbeitsgases.

10. Vorrichtung (10) zur extrakorporalen Blutbehandlung umfassend einen Oxygenator (11) mit einer Hohl-Kapillarmembrane, Mittel zum Zuführen eines venösen Blutstromes mit einem ersten Druckwert (Pᵥ) durch den Oxygenator hindurch und Mittel zum Zuführen eines Arbeitsgasstromes mit einem ersten Druckwert (P1_{g}) durch den Hohlkapillaren, **dadurch gekennzeichnet, dass** sie eine Steuereinheit (19) für die Mittel zum Zuführen eines Arbeitsgasstromes aufweist, welche dazu eingerichtet ist, zumindest einen Druckspitzenwert (P2_{g}) in dem in dem Oxygenator zugeführten Arbeitsgasstrom, unter Einhaltung der Bedingung P1_{g} < P2_{g} < Pᵥ, zu bewirken.

11. Vorrichtung nach Anspruch 10, wobei die Zuführmittel des venösen Blutstroms ihrerseits Mittel zum Erfassen des ersten Druckwertes (Pᵥ) des venösen Blutstromes oder Mittel zum Zuordnen eines konstanten Wertes zu dem Druckwert (Pᵥ) des venösen Blutstromes umfassen.

12. Vorrichtung nach Anspruch 10 oder 11, wobei die Zuführmittel des Arbeitsgasflusses eine Einlassleitung (16) des Arbeitsgases aufweist, die sich von einer externen Quelle (17) in Richtung auf den Hohlkapillaren erstreckt, und die Steuereinheit (19) ein proportionales Elektroventil (20) zum absperren der Einlassleitung aufweist, wobei das Ventil dazu gesteuert werden kann, um zumindest einen Druckspitzenwert (P2g) in dem dem Oxygenator zugeführten Arbeitsgasstrom und somit eine Erhöhung und Erniedrigung der momentanen Flussrate des in den Hohlkapillaren zugeführten Arbeitsgases zu bewirken.

13. Vorrichtung nach Anspruch 12, wobei die Steuereinheit (19) dazu programmiert ist, die Öffnung und Schließung des proportionalen Elektroventils (20) zu steuern, um zumindest einen Druckspitzenwert (P2g) in dem Arbeitsgasfluss in einer Zeitdauer, die kürzer als 3 Sekunden ist, zu bewirken.

14. Vorrichtung nach Anspruch 12 oder 13, wobei die Steuereinheit (19) dazu programmiert ist, die Öffnung und Schließung des proportionalen Elektroventils (20) zu steuern, um eine Vielzahl von Druckspitzenwerten (P2_{g}) in dem Arbeitsgasfluss bei vorgegebenen und/oder programmierbaren Zeitintervallen zu bewirken.

15. Vorrichtung nach einem der vorangehenden Ansprüche 10 bis 14, wobei die Steuereinheit (19) programmierbare Mittel zum Zuordnen eines konstanten Wertes zu dem Druckwert (Pᵥ) des venösen Blutstromes aufweist oder die Mittel zum Erfassen wenigstens einen Sensor aufweisen, der dazu eingerichtet ist, den momentanen Druckwert (Pᵥ) des venösen Blutstromes innerhalb des Oxygenators oder von diesem kommend zu ermitteln.

16. Vorrichtung nach einem der vorangehenden Ansprüche, weiter umfassend zumindest einen Heizer (26) für das Arbeitsgas, der dazu eingerichtet ist, den Temperaturanstieg des Arbeitsgases bis zu einem Wert, der nahe oder gleich dem Temperaturwert des venösen Blutstromes ist, zu bewirken.

17. Vorrichtung nach einem der vorangehenden Ansprüche 10 bis 16, umfassend Mittel zum Aufnehmen der zumindest einer Flussrate des Arbeitsgases, das aus dem Oxygenator nach der Wechselwirkung mit dem Blutstrom herauskommt, und zumindest einen Sensor (30) zum Erfassen der Kohlendioxidkonzentration in dem dem Oxygenator zugeführten Arbeitsgas sowie in dem aus dem Oxygenator herauskommenden Arbeitsgas, und eine Recheneinheit (31)die dazu programmiert ist, die aus dem durch den Oxygenator durchströmten Blutstrom extrahierte Menge von Kohlendioxid als Differenz zwischen den vom Sensor erfassten Werten der Kohlendioxidkonzentration zu berechnen.

## Revendications

1. Procédé d'élimination de la condensation d'un oxygénateur traversé par un flux de sang veineux et pourvu d'une membrane à capillaires creux, le procédé comprenant les étapes de:
a) alimenter un flux de gaz de travail auxdits capillaires creux, à une première valeur de pression (P1_{g}), pour l'élimination du dioxyde de carbone du sang et/ou l'oxygénation de celui-ci;
b) attribuer une valeur constante à la valeur de la pression (Pᵥ) du flux de sang veineux, ou la détecter;
c) produire au moins un pic de pression (P2g) dans le flux de gaz de travail alimenté aux capillaires creux, en prenant soin d'observer la condition P1_{g} <P2_{g} <Pᵥ.

2. Procédé selon la revendication 1, dans lequel ladite valeur de la pression (Pᵥ) du flux veineux est obtenue en détectant la valeur instantanée de la pression du flux du sang veineux dans l'oxygénateur, ou sortant de celui-ci, ou bien ladite valeur de la pression (Pᵥ) du flux veineux est attribuée à une valeur constante.

3. Procédé selon la revendication 2, dans lequel ladite valeur de la pression (Pᵥ) du flux de sang veineux est attribuée à une valeur constante égale à 30 mmHg.

4. Procédé selon l'une quelconque des revendications précédentes, dans lequel ledit pic de pression (P2_{g}) consiste en une augmentation de la pression d'au moins le double par rapport à la première valeur de pression (P1_{g}), suivie d'une diminution de pression, qui redevient égale à la première valeur de pression (P1_{g}), et dans lequel l'augmentation et la diminution de la pression se produisent dans un laps de temps inférieur à 3 s.

5. Procédé selon l'une quelconque des revendications précédentes, dans lequel l'étape c) est obtenue en augmentant et successivement en réduisant le débit instantané du gaz de travail alimenté aux capillaires creux.

6. Procédé selon la revendication 2, dans lequel l'étape c) est effectuée en rétroaction sur la base de la valeur (Pᵥ) détectée de la pression du flux de sang veineux.

7. Procédé selon l'une quelconque des revendications précédentes, dans lequel l'étape c) est effectuée à des intervalles de temps réguliers pendant le traitement hématique extracorporel de l'étape a).

8. Procédé selon l'une quelconque des revendications précédentes, dans lequel, lorsque cela soit nécessaire, avant l'étape a) est prévue l'étape supplémentaire consistant à augmenter la température du gaz de travail, jusqu'à une valeur proche ou égale à la valeur de température du flux de sang veineux.

9. Procédé selon l'une quelconque des revendications précédentes, comprenant les étapes supplémentaires consistant à:
d) détecter la concentration de dioxyde de carbone dans le flux du gaz de travail sortant de l'oxygénateur, et
e) surveiller la quantité de dioxyde de carbone extraite du flux du sang traversant l'oxygénateur en fonction des valeurs de concentration détectées dans l'étape d), multipliées par les valeurs correspondantes du débit du gaz de travail.

10. Appareil (10) pour un traitement hématique extracorporel, comprenant un oxygénateur (11) muni d'une membrane à capillaires creux, des moyens pour alimenter un flux de sang veineux à travers l'oxygénateur à une première valeur de pression (Pᵥ), et des moyens pour alimenter un flux du gaz de travail à travers lesdits capillaires creux à une première valeur de pression (P1_{g}), **caractérisé en ce qu'**il comprend une unité de commande (19) des moyens d'alimentation du gaz de travail programmée pour produire au moins un pic de pression (P2g) dans le flux de gaz de travail alimenté à l'oxygénateur, en respectant la condition P1_{g} < P2_{g} < Pᵥ.

11. Appareil selon la revendication 10, dans lequel lesdits moyens d'alimentation du flux de sang veineux comprennent, à leur tour, des moyens pour détecter la première valeur de la pression (Pᵥ) du flux du sang veineux, ou des moyens pour attribuer une valeur constante à la valeur de la pression (Pᵥ) du flux de sang veineux.

12. Appareil selon la revendication 10 ou 11, dans lequel lesdits moyens d'alimentation du flux du gaz de travail comprennent une ligne d'apport (16) du gaz de travail à partir d'une source externe (17) vers lesdits capillaires creux et l'unité de commande (19) comprend une électrovanne proportionnelle (20) pour intercepter ladite ligne d'apport qui peut être actionnée pour produire ledit au moins un pic de pression (P2_{g}) dans le flux de gaz de travail, en augmentant et en diminuant le débit instantané du gaz de travail alimenté aux capillaires creux.

13. Appareil selon la revendication 12, dans lequel l'unité de commande (19) est programmée pour commander l'ouverture et la fermeture de ladite électrovanne proportionnelle (20) pour produire ledit au moins un pic de pression (P2_{g}) dans le flux de gaz de travail dans un laps de temps inférieure à 3 s.

14. Appareil selon la revendication 12 ou 13, dans lequel l'unité de commande (19) est programmée pour commander l'ouverture et la fermeture de ladite électrovanne proportionnelle (20) pour produire plusieurs pics de pression (P2_{g}) dans le flux de gaz de travail à des intervalles de temps prédéterminés et/ou programmables.

15. Appareil selon l'une quelconque des revendications 10 à 14, dans lequel l'unité de commande (19) comprend des moyens programmables pour attribuer une valeur constante à la valeur de la pression (Pᵥ) du flux de sang veineux, ou bien lesdits moyens de détection comprennent au moins un capteur équipé pour détecter la valeur instantanée de la pression (Pᵥ) du flux de sang veineux à l'intérieur de l'oxygénateur ou sortant de celui-ci.

16. Appareil selon l'une quelconque des revendications, comprenant en outre au moins un élément chauffant (26) du gaz de travail équipé pour augmenter la température du gaz de travail à une valeur proche ou égale à la valeur de la température du flux de sang veineux.

17. Appareil selon l'une quelconque des revendications 10 - 16, comprenant des moyens pour prendre au moins un débit de gaz de travail sortant de l'oxygénateur après son interaction avec le flux de sang, et au moins un capteur (30) destiné à détecter la concentration du dioxyde de carbone dans le gaz de travail alimenté à l'oxygénateur et dans le gaz de travail sortant de l'oxygénateur, et une unité de calcul (31) programmée pour calculer la quantité de dioxyde de carbone extraite du flux de sang qui passe à travers l'oxygénateur comme la différence entre les valeurs de concentration de dioxyde de carbone détectées par ledit capteur.
